# EUROPEAN PATENT SPECIFICATION

(11) **EP 0 550 276 B1**
(45) Date of publication and mention of the grant of the patent: **08.05.1996**
(21) Application number: 92311856.6
(22) Date of filing: 30.12.1992
(51) Int. Cl.: A61K 7/08, C11D 1/37, C11D 1/94, C11D 1/06

(54) **Compositions comprising anionic glycolipid surfactants**
Anionische Glykolipidtenside enthaltende Zusammensetzung
Composition contenant des tensioactifs glycolipidique anioniques

(30) Priority: 31.12.1991 US 816421
(43) Date of publication of application: 07.07.1993
(73) Proprietor: UNILEVER PLC, London EC4P 4BQ (GB); UNILEVER N.V., NL-3013 AL Rotterdam (NL)
(72) Inventor: Gormley, John, Midland Park, New Jersey 07432 (US); Massaro, Michael, Congers, New York 10920 (US); Grudev, George, Hewitt, New Jersey 07421 (US); Vermeer, Robert, Nutley, New Jersey 07110 (US); Harirchian, Bijan, County of Essex, New Jersey (US)
(74) Representative: Fransella, Mary Evelyn

(56) References cited:
- EP-A- 0 242 296
- EP-A- 0 326 673
- EP-A- 0 499 434
- WO-A-90/09451

## Description

### BACKGROUND OF THE INVENTION

The present invention relates to novel personal product or detergent compositions comprising glycolipids, and in particular to compositions comprising anionic derivatives based on uronic acids or lactones as surface active agents in the compositions. Examples of such derivatives include surfactants based galacturonic acid, glucuronic acid, or glucurono-6,3-lactone.

Most surfactants presently used in personal product and detergent compositions are based on petrochemicals. Because of increased concern over environmental issues raised by use of petrochemicals and also because of the continually rising costs of these petrochemicals, it would be useful to develop surfactants which are instead derived from carbohydrates. These natural occurring compounds represent a source of renewable raw materials that are synthetically versatile and environmentally friendly.

The present invention is concerned with carbohydrates, in particular, uronic acid based derivatives, which can be successfully used as mild surfactants in personal product compositions.

Synthesis of pure crystalline uronic acid derivatives is described in DE 3 803 465A (Hüls), equivalent to EP 326 073A. These materials are described as surface-active and suitable for use as detergents or cleansers alone or in combination with other surface-active agents.

EP 242 296A (Petroleum Fermentations) discloses personal care products containing bioemulsifiers, for example, so called "emulsans" which may be regarded as uronic acid derivatives.

### DEFINITION OF THE INVENTION

This invention provides the use as a mild surfactant in personal product compositions of an anionic glycolipid surfactant which is a galacturonic acid derivative having one of the formulae 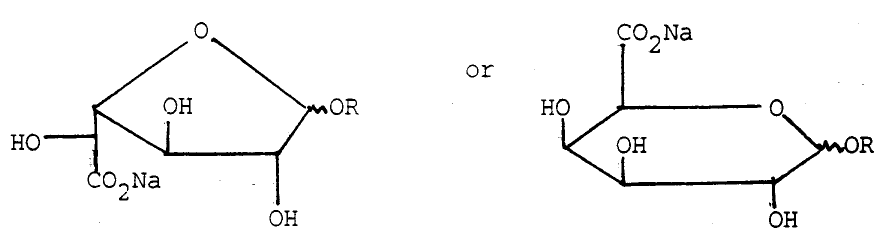
or a glucuronic acid derivative having one of the formulae 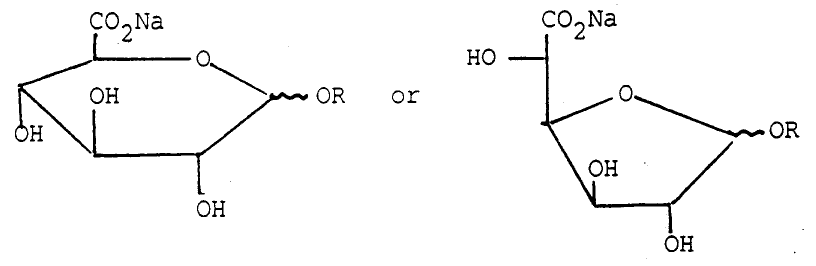
wherein R is a substituted or unsubstituted, saturated or unsaturated, optionally ethoxylated or propoxylated, aliphatic group having 6 to 24 carbon atoms.

### DETAILED DESCRIPTION OF THE INVENTION

The invention is concerned with the use of certain anionic glycolipids - derivatives of galacturonic or glucuronic acid - as mild surfactants in personal product compositions compositions.

Uronic acids have the general formula 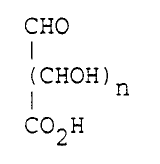
wherein n is an integer of from 1 to 4.

The aldehyde function of the aldose is preserved in the molecules of these compounds while the primary alcohol is replaced by a carboxyl group. From this also follows the chemical behavior of these products, which principally can exist as both pyranoses and furanoses and sometimes lactones. A further description of these types of compounds may be found in the reference "The Monosaccharides" by Jaroslav Stonek et al., p. 695 (1963).

The formulae of the compounds used as mild surfactants in accordance with the present invention are given above. In each case, R is an substituted or unsubstituted, saturated or unsaturated aliphatic group having from 6 to 24, preferably from 10 to 16, carbon atoms. R may also be an alkoxylated alkyl chain (eg ethoxylated or propoxylated). Most preferably, R is an alkyl group having from 10 to 16 carbon atoms.

The uronic acid derived surfactants used in the compositions of the invention have been found to have properties (ie critical micelle concentrations; Krafft Point; foaming; Zein solubilisation indicating that they are equal to or better than other well known anionic surfactants which are based on petrochemicals (for example, sodium dodecyl sulphate) thereby indicating that they can be a viable, environmentally friendly alternative to the use of more traditional anionic surfactants.

In addition, the surfactants of the invention may be used as cosurfactants with other anionic surfactants or with other surfactants (e.g., nonionic, cationic, zwitterionic, amphoteric) used in personal product formulations.

### PERSONAL PRODUCT COMPOSITIONS

Personal product compositions of the invention may be, for example, toilet bar compositions, facial or body cleansing compositions, shampoos for hair or body, conditioners, cosmetic compositions or dental compositions.

In one embodiment of the invention, the uronic acid derived surfactants of the invention may be used, for example, in a toilet bar (i.e. detergent and/or soap bar) formulation.

Typical toilet bar compositions are those comprising fatty acid soaps used in combination with a detergent other than fatty acid soap and free fatty acids. It should be noted that the composition may comprise no fatty acid soap and may be based on actives other than fatty acid soap. Mildness improving salts, such as alkali metal salt or isethionate, are also typically added. In addition other ingredients, such as germicides, perfumes, colorants, pigments, suds-boosting salts and anti-mushing agents may also be added.

Fatty acid soaps are typically alkali metal or alkanol ammonium salts of aliphatic alkane or alkene monocarboxylic acids. Sodium, potassium, mono-, di- and tri-ethanol ammonium cations, or combinations thereof, are suitable for purposes of the invention. The soaps are well known alkali metal salts of natural or synthetic aliphatic (alkanoic or alkenoic) acids having from 8 to 22 carbon atomss, preferably from 12 to 18 carbon atoms.

Examples of soap which may be used may be found in US 4 695 395 (Caswell et al) and US 4 260 507 (Barrett).

In a soap-based bar, fatty acid soaps will generally comprise greater than 25 wt% of the composition, generally from 30-95 wt%. Preferably, the amount of soap will range from 40 to 70 wt% by weight of the composition.

In a bar based on other actives, soap may comprise 0-50% by weight. In general C₈₋₂₄ fatty acid comprises 5-60 wt% of the composition.

The compositions will also generally comprise a non-soap detergent which is generally chosen from anionic, nonionic, cationic, zwitterionic or amphoteric synthetic detergent materials or mixtures thereof. These surfactants are all well known in the art and are described, for example, in US 4 695 395 and US 4 260 507 discussed above. One preferred non-soap anionic is a C₈₋₂₂ alkyl isethionate. These esters may be prepared by the reaction between alkali metal isethionate and mixed aliphatic fatty acids having from 8 to 22 carbons. The non-soap actives may comprise from 0 to 50 wt% of the composition.

A certain amount of free fatty acids of 8 to 22 carbon atoms are also desirably incorporated into soap compositions to act as superfatting agents or as skin feel and creaminess enhancers. If present, the free fatty acids comprise between 1 and 15 wt% of the compositions.

A preferred mildness improving salt which may be added to soap compositions is a simple unsubstituted sodium isethionate. This may be present as 0.1 to 50 wt% of the composition, preferably 0.5 to 25 wt%, more preferably 2 to 15 wt%. Other mildness coactives which may be used include betain compounds or ether sulphates. These also may be present at 0.1 to 50 wt% of the composition, preferably 0.5 to 25 wt%.

The sulphate ester surfactant may comprise 0.01 to 45 wt% by weight of the composition (as the monoester), preferably 25 to 40 wt, and 0.01 to 10 wt% of the composition (as the diester), preferably 0.01 to 5 wt%.

Other optional ingredients which may be present in soap bar compositions are moisturisers such as glycerin, propylene glycol, sorbitol, polyethylene glycol, ethoxylated or methoxylated ether of methyl glucose etc.; water-soluble polymers such as collagens, modified cellulases (such as Polymer JR (Trade Mark)), guar gums and polyacrylates; sequestering agents such as citrate, and emollients such as silicones or mineral oil. Another useful set of ingredients are various cosurfactants and non-soap detergents.

In a second embodiment of the invention, the uronic acid derived surfactant of the invention may be present in a facial or body cleansing composition. Examples of such cleaning compositions are described, for example, in US 4 812 253 (Small et al) and US 4 526 710 (Fujisawa).

Typically, cleansing compositions will comprise a fatty acid soap together with a non-soap surfactant, preferably a mild synthetic surfactant. Cleaning compositions will also generally include a moisturizer or emollient and polymeric skin feel and mildness aids. The compositions may further optionally include thickener (eg magnesium aluminum silicate, Carbopol (Trade Mark)), conditioners, water soluble polymers (eg carboxymethylcellulose), dyes, hydrotropes, brighteners, perfumes and germicides.

The fatty acid soaps used are such as those described above in uses in detergent bar formulations. These soaps are typically alkali metal or alkanol ammonium salts of aliphatic or alkene monocarboxylic salts. Sodium, potassium, mono-, di- and triethanol ammonium cations, or combinations thereof are suitable. Preferred soaps are 8 to 24 carbon half acid salts of, for example, triethanolamine.

Surfactants can be chosen from anionic, nonionic, cationic, zwitterionic or amphoteric materials or mixtures thereof such as are described in US 4 695 395 mentioned above, or in US 4 854 333 (Inman et al).

Moisturisers are included to provide skin conditioning benefits and improve mildness. This term is often used as synonymous with emollient and is then used to describe a material which imparts a smooth and soft feeling to skin surface.

There are two ways of reducing water loss from the stratum corneum. One is to deposit on the surface of the skin an occlusive layer which reduces the rate of evaporation. The second method is to add nonocclusive hydgroscopic substances to the stratum corneum which will retain water, and make this water available to the stratum corneum to alter its physical properties and produce a cosmetically desirable effect. Nonocclusive moisturisers also function by improving the lubricity of the skin.

Both occlusive and nonocclusive moisturisers can work in the present invention. Some examples of moisturisers are long chain fatty acids, liquid water-soluble polyols, glycerin, propylene glycol, sorbitol, polyethylene glycol, ethoxylated/propoxylated ethers of methyl glucose (eg., methyl gluceth-20) and ethoxylated/-propoxylated ethers of lanolin alcohol (eg Solulan-75).

Preferred moisturisers are coco and tallow fatty acids. Some other preferred moisturisers are the nonocclusive liquid water soluble polyols and the essential amino acid compounds found naturally in the skin.

Other preferred nonocclusive moisturisers are compounds found to be naturally occurring in the stratum corneum of the skin, such as sodium pyrrolidone carboxylic acid, lactic acid, urea, L-proline, guanidine and pyrrolidone. Examples of other nonocclusive moisturisers include hexadecyl, myristyl, isodecyl or isopropyl esters of adipic, lactic, oleic, stearic, isostearic, myristic or linoleic acids, as well as many of their corresponding alcohol esters (sodium isostearoyl-2 lactylate, sodium capryl lactylate), hydrolyzed protein and other collagen-derived proteins, aloe vera gel and acetamide MEA.

Some occlusive moisturisers include petrolatum, mineral oil, beeswax, silicones, lanolin and oil-soluble lanolin derivatives, saturated and unsaturated fatty alcohols such as behenyl alcohol, squalene and squalane, and various animal and vegetable oils such as almond oil, peanut oil, wheat germ oil, linseed oil, jojoba oil, oil of apricot pits, walnuts, palm nuts, pistachio nuts, sesame seeds, rapeseed, cade oil,corn oil, peach pit oil, poppyseed oil, pine oil, castor oil, soybean oil, avocado oil, safflower oil, coconut oil, hazelnut oil, olive oil, grape seed oil and sunflower seed oil.

Other examples of both types of moisturisers are disclosed in "Emollients -- a Critical Evaluation," by J. Mausner, Cosmetics & Toiletries, May 1981.

The polymeric skin feel and mildness aids useful in the present invention are the cationic, anionic, amphoteric, and the nonionic polymers used in the cosmetic field. Reduced skin irritation benefits as measured by patch testing of cationic and nonionic types of polymers are set out in "Polymer JR for Skin Care" Bulletin, by Union Carbide, 1977. The cationics are preferred over the others because they provide better skin feel benefits.

The amount of polymeric skin feel and mildness aids found useful in the composition of the present invention is from 0.01 to 5 wt%, preferably from 0.3 to 4 wt%. In bar compositions with less than 5.5 wt% soap, the polymer is suitably used at a level of 2 to 5 wt%, preferably 3 wt% or more.

Other types of high molecular weight polymeric skin feel and skin mildness aids, such as nonionic guar gums, Merquats (Trade Mark) 100 and 550, made by Merck & Co, Inc.; Jaguar (Trade Mark) C-14-S made by Stein Hall; Mirapol (Trade Mark) A15 made by Miranol Chemical Company, Inc.; and Galactasol (Trade Mark) 811, made by Henkel, Inc.; plus others, are usable. The polymer also provides enhanced creamy lather benefits.

Nonionic polymers found to be useful include the nonionic polysaccharides, e.g., nonionic hydroxypropyl guar gums, offered by Celanese Corp. A preferred nonionic hydroxypropyl guar gum material is Jaguar (Trade Mark) HP-60 having molar substitution of about 0.6. Another class of useful nonionics is the cellulosic nonionic polymers, for example, HEC and CMC.

The cationic polymers employed in this invention also provide a desirable silky, soft, smooth in-use feeling. The preferred level for this invention is 0.1-5 wt% of the composition. There is reason to believe that the positively charged cationic polymers can bind with negatively charges sites on the skin to provide a soft skin feel after use. Not to be bound by any theory, it is believed that the greater the charge density of the cationic polymer, the more effective it is for skin feel benefits.

Other suitable cationic polymers are copolymers of dimethylaminoethylmethacrylate and acrylamide and copolymers of dimethyldiallylammonium chloride and acrylamide in which the ratio of the cationic to neutral monomer units has been selected to give a copolymer having a cationic charge. Yet other suitable types of cationic polymers are the cationic starches, for example, Sta-Lok (Trade Mark) 300 and 400 made by, Staley, Inc.

A more complete list of cationic polymers useful in the present invention is described in US 4 438 095 (Grollier/allec). Some of the more preferred cationics are listed in Col. 3, Section 2; Col. 5, section 8; Col. 8, section 10; and Col. 9, lines 10-15 of the Grollier/allec patent,

In a third embodiment of the invention, the uronic acid derived surfactant of the invention may be used, for example, in a hair or body shampoo. Examples of such compositions are described in US 4 854 333 (Inman) and US 4 526 710 (Fujisawa).

The shampoo compositions which may be used typically comprise a surfactant selected from any one of a wide variety of surfactants known in the art (such as those described in US 4 854 333, incorporated herein by reference). The shampoo compositions may additionally comprise a compound considered useful for treating dandruff, e.g. selenium sulphide.

The compositions all may also optionally comprise a suspending agent, for example, any of several acyl derivative materials or mixtures thereof. Among these are ethylene glycol esters of fatty acids having 16 to 22 carbons. Preferred suspending agents include ethylene glycol stearates, both mono-and distearate. Preferred alkanol amides are stearic monoethanolamide, stearic diethanolamide and stearic monoisopropanolamide. Still other long chain acyl derivatives include long chain esters of long chain fatty acids (for example, stearyl stearate, cetyl palmitate), glyceryl esters (for example, glyceryl distearate), and long chain esters of long chain alkanol amides (for example, stearamide DEA distearate, stearamide MEA stearate).

Still other suitable suspending agents are alkyl (16 to 22 carbon) dimethyl amine oxides, such as stearyl dimethyl amine oxide. If the compositions contain an amine oxide or a long chain acyl derivative as a surfactant, these components may also provide the suspending function and additional suspending agent may not be needed.

Xanthan gum is another agent used to suspend, for example, selenium sulphide which may be in the present compositions. This biosynthetic gum material is commercially available and is a heteropolysaccharide with a molecular weight of greater than 1 million. It is believed to contain D-glucose, D-mannose and D-glucuronate in the molar ratio of 2.8:2.0:2.0. The polysaccharide is partially acetylated with 4.7% acetyl. Supplemental information on these agents is found in Whistler, Roy L. (Editor), Industrial Gums --Polysaccharides and Their Derivatives, Academic Press, New York, 1973. Kelco, a Division of Merck & Co., Inc., offers xanthan gum as Keltrol (Trade Mark).

A particularly preferred suspending system comprises a mixture of xanthan gum, present at a level of from 0.05 to 1.0 wt%, preferably from 0.2 to 0.4 wt%, of the compositions, together with magnesium aluminum silicate (Al₂Mg₈Si₂), present at a level of from 0.1 to 3.0 wt%, preferably from 0.5 to 2.0 wt%, of the compositions. Magnesium aluminum silicate occurs naturally in such smectite minerals as colerainite, saponite and sapphire. Refined magnesium aluminum silicates useful herein are readily available, for example as veegum, manufactured by RT Vanderbilt Company, Inc. Mixtures of suspending agents are also suitable for use in the compositions of this invention.

Other useful thickening agents are the cross-linked polyacrylates such as those manufactured by B F Goodrich and sold under the name Carbopol (Trade Mark).

Another optional component for use in the present compositions is an amide. The amide used in the present compositions can be any of the alkanolamides of fatty acids known for use in shampoos. These are generally mono- and diethanolamides of fatty acids having from 8 to 24 carbon atoms. Preferred are coconut monoethanolamide, lauric diethanolamide and mixtures thereof. The amide is suitably present at a level of from 1 to 10 wt% of the compositions.

The compositions may also contain nonionic polymer material which is used at a low level to aid in dispersing particles. The material can be any of a large variety of types including cellulosic materials such as hydroxypropyl methyl cellulose, carboxymethyl cellulose, hydroxyethyl cellulose and sodium carboxymethyl cellulose as well as mixtures of these materials. Other materials include alginates, polyacrylic acids, polyethylene glycol and starches, among many others. The nonionic polymers are discussed in detail in Industrial Gums, edited by Roy L Whistler, Academic Press Inc., 1973, and Handbook of Water-Soluble Gums and Resins, edited by Robert L Davidson, McGraw-Hill Inc., 1980.

When included, the nonionic polymer is suitably used at a level of from 0.001 to 0.1 wt%, preferably from 0.002 to 0.05 wt%, of the composition. Hydroxypropyl methyl cellulose is the preferred polymer.

Another suitable optional component useful in the present compositions is a nonvolatile silicone fluid.

The nonvolatile silicone fluid may be either a polyalkyl siloxane, a polyaryl siloxane, a polyalkylarly siloxane or a polyether siloxane copolymer and is suitably present at a level of from 0.1 to 10.0 wt%, preferably from 0.5% to 5.0 wt%. Mixtures of these fluids may also be used and are preferred in certain executions. The dispersed silicone particles should also be insoluble in the shampoo matrix. This is the meaning of "insoluble" as used herein.

The essentially nonvolatile polyalkyl siloxane fluids that may be used include, for example, polydimethyl siloxanes with viscosities ranging from about 5 to about 600 000 centistokes at 25°C. These siloxanes are available, for example, from the General Electric Company as the Viscasil series and from Dow Corning as the Dow Corning 200 series The siloxane viscosity can be measured by means of a glass capillary viscometer as set forth in Dow Corning Corporate Test Method CTM0004, July 20, 1970. Preferably the viscosity of the these siloxanes range from about 350 centistokes to about 100 000 centistokes.

The essentially nonvolatile polyether siloxane copolymer that may be used is, for example, a polypropylene oxide modified dimethylpolysiloxane (for example, Dow Corning DC-1248), although ethylene oxide or mixtures of ethylene oxide and propylene oxide may also be used.

Suitable silicone fluids are described in US 2 826 551 (Geen), US 3 946 500 (Drakoff), US 4 364 837 (Pader) and GB 849 433 (Woolston). Also incorporated herein by reference is Silicon Compounds, distributed by Petrarch Systems Inc., 1984. This reference provides a very good listing of suitable silicone materials.

Another silicone material useful is silicone gum. Silicone gums are described by Petrarch and others including US 4 152 416 (Spitzer et al), and Noll, Chemistry and Technology of Silicones, Academic Press, New York, 1968. Useful silicone gums are also described in General Electric Silicone Rubber Product Data Sheets SE 30, SE 33, SE 54 and SE 76. All of these references are incorporated herein by reference "Silicone gum" materials denote high molecular weight polydiorganosiloxanes having a mass molecular weight of from about 200 000 to about 1 000 000. Specific examples include polydimethylsiloxane, (polydimethylsiloxane) (methylvinylsiloxane) copolymer, poly(dimethylsiloxane) (diphenyl) (methylvinylsiloxane) copolymer, and mixtures thereof. Mixtures of silicone fluids and silicone gums are also useful herein.

The shampoos herein can contain a variety of other nonessential optional components suitable for rendering such compositions more formulatable, or aesthetically and/or cosmetically acceptable. Such conventional optional ingredients are well-known to those skilled in the art and include, for example, preservatives, such as benzyl alcohol, methyl paraben, propyl paraben, and imidazolinidyl urea; cationic surfactants, such as cetyl trimethyl ammonium chloride, lauryl trimethyl ammonium chloride, tricetyl methyl ammonium chloride, stearyldimethyl benzyl ammonium chloride, and di(partially hydrogenated tallow) dimethylammonium chloride; menthol; thickeners and viscosity modifiers, such as block polymers of ethylene oxide and propylene oxide such as Pluronic (Trade Mark) F88 offered by BASF Wyandotte, sodium chloride, sodium sulphate, propylene glycol, and ethyl alcohol; pH adjusting agents, such as citric acid, succinic acid, phosphoric acid, sodium hydroxide, sodium carbonate; perfumes; dyes; and sequestering agents, such as disodium ethylenediamine tetraacetate. Such agents generally are used individually at a level of from 0.01 to 10 wt%, preferably from 0.5 to 5.0 wt%, of the composition.

A typical shampoo composition might comprise (percentages by weight):
(1) 5-15% uronic acid derivative
(2) 0-10% anionic coactive
(3) 0-10% amphoteric coactive
(4) 0-5% lauramide MEA
(5) 0-5% thickener
(6) 0-2% fragrance
(7) 0-1% preservative; and
(8) remainder water
In a fourth embodiment of the invention, the uronic acid derived surfactant of the invention may be used in a conditioner composition such as is taught and described in US 4 913 828 (Caswell et al).

More particularly, conditioner compositions are those containing a conditioning agent (e.g. alkylamine compounds) such as those described in US 4 913 828.

In a fifth embodiment of the invention, the surfactant may be used in a cosmetic composition, such as is taught and is described in EP 371 803A.

Such compositions generally comprise thickening agents, preservatives and further additions.

The composition may comprise polymer thickener in an amount sufficient to adjust the viscosity of the composition, so as to facilitate dispensing it conveniently onto the body surface.

Examples of polymer thickeners include: anionic cellulose materials, such as sodium carboxy methyl cellulose; anionic polymers such as carboxy vinyl polymers, for example, Carbomer (Trade Mark) 940 and 941; nonionic cellulose materials, such as methyl cellulose and hydroxy propyl methyl cellulose; cationic cellulose materials, such as Polymer JR (Trade MarkO 400; cationic gum materials, such as Jaguar (Trade Mark) C13 S; other gum materials such as gum acacia, gum tragacanth, locust bean gum, guar gum and carrageenan; proteins, such as albumin and protein hydrolysates; and clay materials, such as bentonite, hectorite, magnesium aluminum silicate, or sodium magnesium silicate.

Generally, the thickening agent may comprise from 0.05 to 5 wt%, preferably 0.1 to 1% by weight of the composition.

The composition according to the invention can also optionally comprise a preservative to prevent microbial spoilage.

Examples of preservatives include:
(i) Chemical preservatives, such as ethanol, benzoic acid, sodium benzoate, sorbic acid, potassium sorbate, sodium propionate and the methyl, ethyl, propyl and butyl esters of p-hydroxybenzoic acid 2-bromo-2-nitropropane-1, 3-diol, phenoxyethanol, dibromodicyanobutane, formalin and Triclosan (Trade Mark). The amount of chemical preservative optionally to be incorporated in the composition according to the invention will generally be from 0.05 to 5 wt%, preferably from 0.01 to 2% by weight, the amount chosen being sufficient to arrest microbial proliferation.
(ii) Water activity depressants, such as glycerol, propylene glycol, sorbitol, sugars and salts, for examples alkali metal halides, sulphates and carboxylates. When employing a water activity depressant, sufficient should be incorporated in the composition according to the invention to reduce the water activity from 1 to <0.9, preferably to <0.85 and most preferably <0.8, the lowest of these values being that at which yeasts, moulds and fungi will not proliferate.

The composition can also contain other optional adjuncts, which are conventionally employed in compositions for topical application to human skin. These adjuncts, when present, will normally form the balance of the composition.

Examples of optional adjuncts include vehicles, the selection of which will depend on the required product form of the composition. Typically, the vehicle when present, will be chosen from diluents, dispersants or carriers for the dialkyl or dialkenyl phosphate salt so as to ensure an even distribution of it when applied to the skin.

Compositions according to this invention can include water as a vehicle, usually with at least one other cosmetically-acceptable vehicle.

Vehicles other than water that can be used in compositions according to the invention can include liquids or solids as emollients, solvents, humectants, thickeners and powders. Examples of each of these types of vehicles, which can be used singly or as mixtures of one or more vehicles, are as follows:
emollients, such as stearyl alcohol, glyceryl monolaurate, glyceryl monoricinoleate, glyceryl monostearate, propane-1, 2-diol, butane-1.3 diol, docosan-1,2-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, soybean oil, sunflower seed oil, olive oil, sesame seed oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;
propellants, such as trichlorofluoromethane, dichlorodifluoromethane, dichlorotetrafluoromethane, monochlorodifluoromethane, trichlorotrifluoromethane, propane, butane, isobutane, dimethyl ether, carbon dioxide, nitrous oxide;
solvents, such as ethyl alcohol, methylene chloride, isopropanol, acetone, castor oil, ethylene glycol monoethyl ether, diethylene glycol monobutyl ether, diethylene glycol monoethyl ether, dimethyl sulphoxide, dimethyl formamide, tetrahydrofuran,
humectants, such as glycerin, sorbitol, sodium 2-pyrrolidone-5-carboxylate, soluble collagen, dibutyl phthalate, gelatin;
powders, such as chalk, talc, fuller's earth, kaolin, starch, gums, colloidal silicon dioxide, sodium polyacrylate, tetra alkyl and/or trialkyl aryl ammonium smectites, chemically modified magnesium aluminum silicate, organically modified montmorillonite clay, hydrated aluminum silicate, fumed silica, carboxyvinyl polymer, sodium carboxymethyl cellulose, ethylene glycol monostearate.

The cosmetically acceptable vehicle, when present, will usually form from 0.01 to 99.9 wt%, preferably from 59 to 98% by weight of the composition, and can, in the absence of other cosmetic adjuncts, form the balance of the composition.

A wide variety of conventional sunscreening agents, such as those described in US 4 919 934 (Deckner et al). may also be used in the cosmetic compositions of the invention.

Such agents include, for example, p-aminobenzoic acid, its salts and its derivatives, anthranilates, salicylates, cinnamic acid derivatives, di- and trihydroxy cinnamic acid derivatives, hydrocarbons such as diphenylbutadiene and stilbene, dibenzalacetone and benzalacetophenone, naphthasulphonates, di-hydroxy naphthloic acid and its salts, hydroxy diphenylsulphonates, coumarin derivatives, diazoles, quinine salts, quinoline derivatives, hydroxy or methoxy substituted benzophenones, uric or vilouric acid, tannic acid and its derivatives, hydroquinone, and benzophenones.

In a sixth embodiment of the invention, the surfactant may be used in a toothpaste composition such as is taught and is described in US 4 935 227 (Duckworth).

Such compositions generally comprise abrasive gels (e.g. calcium carbonate), oral therapeutic agents (for example, fluorine containing compound), coactives, flavoring agents, sweetening agents, humectants and binding or thickening gels.

Preferred toothpastes of this invention comprise 0 to 1.5 wt% by weight of anionic surfactant. In more preferred products the amount of anionic surfactant is 0 to 1% by weight with most preferred amounts being 0 to 0.75% by weight.

Toothpastes of this invention may include other surfactants, especially nonionic surfactants.

Toothpaste of the invention will also comprise the usual additional ingredients in particular humectant binder or thickening agent.

Humectants which may be used include glycerol, sorbitol syrup, polyethylene glycol, lactitol, xylitol or hydrogenated corn syrup. The total amount of humectant present will generally range from 10 to 85% by weight of the toothpaste.

Numerous binding or thickening agents have been indicated for use in toothpastes, preferred ones being sodium carboxymethylcellulose, cross-linked polyacrylates and xanthan gum. Others include natural gum binders such as gum tragacanth, gum karaya and gum arabic, Irish moss, alginates, and carrageenans. Silica thickening agents include the silica aerogels and various precipitated silicas. Mixtures of binders and thickeners may be used. The amount of binder and thickening agent included in a toothpaste is generally from 0.1 to 15% by weight.

In a seventh embodiment of the invention the surfactant of the invention may be used in underarm deodorant/antiperspirant compositions such as those taught in US 4 919 934 (Deckner), US 4 944 937 (McCall) and US 4 944 938 (Patini).

Such compositions generally comprise a cosmetic stick (gel or wax) composition which in turn generally comprises one or more liquid base materials (for example, water, fatty acid and fatty alcohol esters, water-insoluble ethers and alcohols, polyorganosiloxanes); a solidifying agent for solidifying the liquid base; and an active component such as bacteriostats or fungistats (for anti-deodorant activity) or astringent metallic salts (for antiperspirant activity).

These compositions may also comprise hardeners, strengtheners, emollients, colorants, perfumes, emulsifiers and fillers.

While various compositions are described above, these should not be understood to be limiting as to what other personal product compositions may be used since other compositions which may be known to those of ordinary skill in the art are also contemplated by this invention.

The invention is set forth in greater detail in the examples which follow below. These examples are merely to illustrate the invention and are not intended to be limiting in any way.

### EXAMPLE 1

### Experimental

### Synthesis of Sodium(Alkyl D-Galactosid)Uronates (SAGAL) - General Procedure

D-Galacturonic acid can undergo direct esterification and glycosylation with various linear alcohols (C₁₀-C₁₆) in the presence of an emulsifier such as alkyl polyglycoside (APG) or dialkylgalactosid uronates. Subsequent alkaline hydrolysis of the intermediate affords the corresponding product as sugar-based anionic surfactants. An example of this reaction as well as the overall yields is shown as follows:

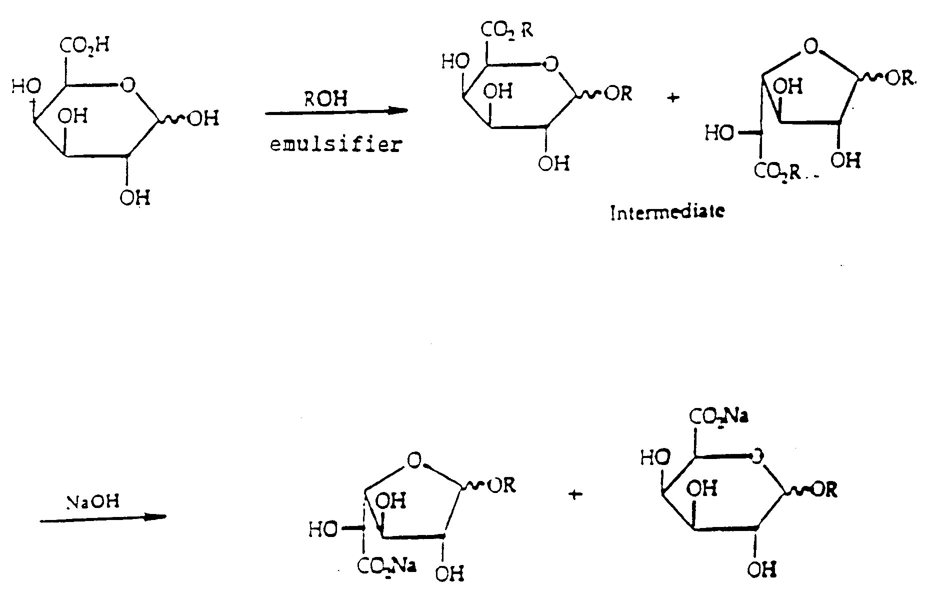

**Table 1**

| Entry | R | Overall Yield (α + β Anomers) |
|---|---|---|
| 1 | C₁₀ | 82.3% |
| 2 | C₁₂ | 81.7% |
| 3 | C₁₄ | 78.4% |
| 4 | C₁₆ | 80.0% |

The " 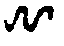 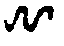 " corresponds to α and β anomers

This reaction indicates that SAGAL salt derivatives contain the furanose and pyranose ring structure.

Examination of Table 1 indicates that salts can be prepared in good yield (78-82%) form various linear alcohols (R = C₁₀-C₁₆) in the presence of an emulsifier.

In addition to synthesis using an emulsifier such as APG (exemplified in examples 2-4) or dialkylgalactoside uronates, the surfactants of the invention may also be made using an ROH in combination with acid (for example, a mono or polycarboxylic acid, mono or polysulphonic acid, HCl, H₂SO₄, etc.) and a lower alcohol (See Examples 5 and 6). One example of this type of procedure is described in US 4 465 828 (Rau et al).

Finally, the surfactants of the invention may be made using alcohol ROH with acid alone (see Example 7).

### EXAMPLE 2

### The Preparation of Decyl D-Galactopyranurate Sodium Salt (AGU Salt)

20.0 g (0.094 moles) of 97% D-galacturonic acid monohydrate in 446 g (0.282 moles) of 1-decanol was placed in a four-necked bottom flask equipped with a mechanical stirrer, thermometer and short path distillation head. 4-0 g (10% by weight based on D-galacturonic acid monohydrate) of APG 300 glycoside (Horizon Chemical) was added rapidly. The mixture was heated for 5 hours at 90-100°C with rapid agitation. 3.4 g (0.188 moles) of water under 130 mm Hg vacuum were removed to a maximum temperature of 95°C. After cooling to 30°C, the crude product was checked by NMR (anomeric H at 4.8-4.9 ppm) and by IR (ester carbonyl at 1741 cm-1). The pH of the reaction mixture was adjusted to 9.5 with 3.8 g (0.094 moles) of sodium hydroxide in 35 ml of distilled water. The mixture was then heated to 60-70°C maintaining a pH of 9.5 for 2 hours. Approximately 25 ml of water were removed under 85 mm Hg vacuum to a maximum temperature of 80°C. 9.5 g (0.060 moles) of 1-decanol were removed by slowly increasing the vacuum to 0.1 mm Hg at a maximum temperature of 110°C.

The resulting paste was cooled to 30°C and slurried with 150 ml acetone; heated to reflux for 0.5 hours; filtered while warm; and the product collected onto a Buchner funnel. The product was washed three times with 50 ml of warm acetone, reslurried, and washed as above until the filtrate remained clear. Trace amounts of acetone were removed under 0.1 mm Hg vacuum.

The product was characterised as follows:
¹H NMR (200 MHz ppm D2O/TSP) 0.88 (bt), 1.3 (s), 1.6 (bq), 3.3 - 4.5 (m), and 4.9 (s).
IR (Nujol Mull cm-1) 3400 (OH), 2953, 2922, 2853, 1460, 1376, (CH2, CH3), 1612, 1411 (RCOONa), and 1090-1020 (CO).
¹³C NMR (200 MHz D2O/TSP) 180.5, 177.8, 110.1, 103.4, 87.1, 83.6, 79.7, 78.8, 74.4, 71.0, 34.6, 32.4, 32.2, 32 0, 31.9, 28.6, 25.2, 16.4.
TSP/MS (60/40 CH3CN/H2O in 0.05 M CH3CO2NH4 buffer) 335.3 352.3, 357.2, 379.2, 380.3, 461.2, 543.2. (All units mass/charge).

### EXAMPLE 3

### Preparation of Sodium(dodecyl D-Galactosid)uronates (SC12 GAL) using APG emulsifier technique

This was prepared according to the procedure described for SC10 GAL salt except 1-dodecanol was used. The yield was 81.7%.

### EXAMPLE 4

### Preparation of Sodium(tetradecyl D-galactosid) and Sodium (hexadecyl D-Galactosid)uronates using APG emulsifier technique

These were prepared according to the procedure described for the SC10 GAL salt except excess alcohol was removed from the reaction mixture after pH adjustment by washing with warm acetone instead of distillation. The yields of C₁₄ and C₁₆ salts were 78.4% and 80.0% respectively.

### EXAMPLE 5

### Preparation of Sodium(tetradecyl D-glucosid)uronate (SC14GLU) using acid and alcohol technique

D-Glucurono-6,3-lactone (623.4g, 3.54 moles), about 0.15 moles of p-toluenesulphonic acid and 1-butanol (1.31kg, 17.7 moles) were placed in a 12 litre three-neck flask equipped with a mechanical stirrer, thermometer and short path distillation head. The mixture was heated for 2-4 hrs. at 80-85°C until all had dissolved. 1-Tetradecanol (1.67kg, 7.80 moles) was added to the reaction mixture and a gentle vacuum applied to remove the water and butanol. The vacuum was increased in increments over a four hour period while maintaining a temperature of 75-85°C. The vacuum was removed when ∼ 2.00 liters of 1-butanol and about 64 ml of water were collected. Acetone (4 litres) was added to the cooled (20°C) product along with sodium hydroxide solution (273g, 26.8N). The dark precipitating product was allowed to stir at room temperature for 16 hrs. The crude product was filtered on a fritted funnel 70-100,u and washed with acetone (3x 1500ml).

The remaining 1-tetradecanol was removed by Soxhlet extraction in 500g batches (crude weight) with acetone or by starring (100g batch) in warm acetone (1 litre) for 3 hrs followed by filtration through a 70-100u glass fritted funnel. After filtering, the product was washed with acetone (3x 1500ml). The colour of the product was removed by slurring with acetone (4000 ml) containing 30% hydrogen peroxide (25 ml) at room temperature. Additional amounts of 30% peroxide were added in 25 ml batches as required after test paper monitoring showed the disappearance of peroxide. A total of 125 ml of 30% hydrogen peroxide was added over 96 hours. The product was filtered and washed with acetone (2x 1000ml). The overall yield was 841.2g (57.70%), the isolated yield was 741.9g (50.87%) based on 88.2% purity.

### EXAMPLE 6

### Preparation of Sodium(tetradecyl D-galactosid)uronate (SC14GAL) using acid and alcohol technique

D-Galacturonic acid monohydrate (750g, 3.54 moles), p-toluenesulphonic acid (26.5g, 0.14 mole), and 1-butanol (1.31kg, 17.7 moles) were placed in a 12 litre three-neck flask equipped with a mechanical stirrer, thermometer and short path distillation head. The mixture was heated 4 hrs. at 80-85°C until all had dissolved. 1-Tetradecanol (1.67kg, 7.80 moles) was added to the reaction mixture and a gentle vacuum applied to remove the water and butanol. The vacuum was increased in increments over an eight hour period while maintaining a temperature of 85-90°C. The vacuum was removed when ∼2.00 litres of 1-butanol and ∼191 ml of water were collected. Acetone (4 litres) was added to the cooled (20°C) product along with sodium hydroxide (152.3g) in water (137.2g). The precipitating product was allowed to stir at room temperature for 67 hrs. The crude product was filtered on a fritted funnel of medium porosity and washed with acetone (3x 2000 ml).

The remaining 1-tetradecanol was removed by Soxhlet extraction in 500g batches with acetone for 192 hrs. The yellow product was slurried with acetone (2500 ml) and bleached with 30% hydrogen peroxide (50 ml) at room temperature for 96 hrs. The white product was filtered on a fritted funnel of medium porosity and washed with acetone (2x 1500 ml). The overall yield was 1226g (83.95%), the isolated yield was 1048g (71.77%) based on 85.5% purity.

### EXAMPLE 7

### Preparation of Sodium(butyl D-glucosid)uronate using acid and alcohol technique

This is prepared on a 10g scale according to the procedure described for SC14 GLU except 1-butanol is used exclusively instead of 1-tetradecanol and a vacuum distillation is started immediately after all has dissolved.

### EXAMPLE 8

### Preparation of Sodium(butyl D-galactosid)uronate using acid and alcohol technique

This is prepared on a 10g scale according to the procedure described for SC14 GAL except 1-butanol is used exclusively instead of 1-tetradecanol and a vacuum distillation is started immediately after all has dissolved.

### EXAMPLE 9

### Preparation of Sodium(decyl D-galactosid)uronate (SC10Gal) (Direct method)

This is prepared on a 10g scale according to the procedure described for SC10GAL except 1-butanol is not used and a vacuum distillation is started immediately to remove water after the starting materials are dissolved.

### SURFACTANCY

In order to determine the effectiveness of these compounds as surfactants, various physical properties (i.e., CMC, Krafft point, foam height, Zein dissolution) of the surfactant, which are associated with the "surfactancy" of each molecule, were measured. In particular, the properties were compared to well known and commonly used anionic surfactant sodium dodecyl sulphonate (SDS). The results of which various measurements are set forth in Examples 10 to 12 below.

### Critical Micelle Concentration (CMC)

The CMC is defined as the concentration of a surfactant at which it begins to form micelles in solution. Specifically, materials that contain both a hydrophobic group and a hydrophilic group (such as surfactants) will tend to distort the structure of the solvent (ie water) they are in and therefore increase the free energy of the system. They therefore concentrate at the surface, where, by orienting so that their hydrophobic groups are directed away from the solvent, the free energy of the solution is minimised. Another means of minimising the free energy can be achieved by the aggregation of these surface-active molecules into clusters or micelles with their hydrophobic groups directed toward the interior of the cluster and their hydrophilic groups directed toward the interior of the cluster and their hydrophilic groups directed toward the solvent.

The value of the CMC is determined by surface tension measurements using the Wilhemy plate method.
While not wishing to be bound by theory, it is believed that a low CMC is a measure of surface activity (i.e., lower CMC of one surfactant versus another indicates the surfactant with lower CMC is more surface active). In this regard, it is believed that lower CMC signifies that lesser amounts of a surfactant are required to provide the same surfactancy benefits as a surfactant with higher CMC.

The CMC of various surfactants were measured and the results set forth below:

### Critical Micelle Concentration (CMC) Measurements on Galacturonic Salt Derivatives

| Surfactant | CMC |
|---|---|
| SDS | 8 mM |
| SC10GAL | 1.5-2.0 mM |
| SC12GAL | 0.5-1.0 mM |
| SC14GAL | --- |
| SC16GAL | --- |

Specifically, the CMC was determined by plotting surface tension as, a function of log (concentration) and extrapolating linear points to obtain an intersection point. The concentration at this point is taken as the CMC.

As can be seen from the table above, the CMC value for the galacturonic acid salts are significantly lower than SDS. These numbers indicate that it would be expected for the surfactants to have as good or as better surfactancy than SDS, a well-known anionic surfactant, in this regard.

### EXAMPLE 10: Krafft Points

The temperature at and above which surfactants begin to form micelles instead of precipitates is referred to as Krafft point (Tₖ) and at this temperature the solubility of a surfactant becomes equal to its CMC.

Krafft point was measured by preparing a 1 wt% dispersion of the surfactant in water. If the surfactant was soluble at room temperature, the solution was cooled to 0°C. When the surfactant did not precipitate out, its Krafft point was considered to be <0°C. If it precipitated out, the solution was slowly warmed with stirring in a water bath. The temperature at which the precipitate dissolved was determined to be the Krafft point.

If the Krafft point was above room temperature, the solution was first heated rapidly to dissolve all the surfactant It was then cooled until precipitation occurred, and was then slowly warmed to determine the Krafft point described above.

While not wishing to be bound by theory, it is believed that lower Krafft points are indicative of a surfactant being more soluble in aqueous system. Also, since micelles exist only at temperature above Tₖ, surfactants with high Tₖ will show lower activity at low temperatures.

The Krafft point of various galacturonic salts are set forth as follows:

### Krafft Point Measurement on Galacturonic Salt Derivatives

| Surfactant | Krafft Point (Tₖ) |
|---|---|
| SDS | 16°C |
| SC10 GAL | 0°C |
| SC12 GAL | 0°C |
| SC14 GAL | 0°C |
| SC16 GAL | 41°C |

As seen from the table above, the low Krafft point temperature for most derivatives indicates that they will be highly soluble in aqueous systems and that the derivatives will have higher surfactant activity at lower temperatures (ie since micelles will form at lower temperatures).

### EXAMPLE 11: Foam Height

Foam is an important attribute in many consumer products Foam is one of the dominant factors that determines the commercial value of products such as shampoo, soap, etc. Also, acceptability of many consumer products is closely related to the quality and texture of the foam they produce (psychological aspect).

Since most of the foaming data on surfactants is typically obtained by the Ross-Miles method (Ross, J. and Miles, G.D., Am. Soc. for Testing Material Method D1173-53 Philadelphia, PA. [1953]; Oil & Soap [1958] 62:1260) the foaming ability of these surfactants was also acquired using this method.

In the Ross-Miles method, 200 ml of a solution of surfactant contained in a pipette of specified dimensions with a 2.9-mm-i.d. orifice is allowed to fall 90 cm onto 50 ml of the same solution contained in a cylindrical vessel maintained at a given temperature (often 60°C) by means of a water jacket. The height of the foam produced in the cylindrical vessel is read immediately after all the solution has run out of the pipette (initial foam height) and then again after a given amount of time (generally, 5 min).

Using this method, the foam production (measured initially) and foam stability (the height after 10 minutes) are reported. All of the foaming was achieved at 45°C in water with 120 ppm hardness. The foam height is represented in millimetres (mm).

The initial foam height and height after 10 minutes (i.e. foam stability) is measured at 40°C for various galacturonic salts and SDS. Measurements were at 0.05 wt% and 0.10 wt% surfactant concentration and results are set forth below:

| | 0.05% CONCENTRATION | | 0.10% CONCENTRATION | |
|---|---|---|---|---|
| | Initial Height | After 10 Minutes | Initial Height | After 10 Minutes |
| SDS | 143 | 128 | 160 | 150 |
| SC10 GAL | 105 | 99 | 125 | 119 |
| SC12 GAL | 126 | 109 | 155 | 139 |
| SC14 GAL | 143 | 129 | 160 | 148 |
| SC16 GAL | 123 | 119 | 123 | 118 |

As can be seen from above, foam height was comparable to SDS in most cases and most nearly comparable at SC14 GAL. Thus, the surfactant provides adequate foaming benefits relative to other anionics.

### EXAMPLE 12: Zein Test

### In vitro "Mildness" Test Assessing Mildness

Many factors have been reported to have an influence on skin irritation such as removal of skin lipids, loss of naturally occurring hygroscopic materials in the stratum corneum, adsorption, protein denaturation, and epidermal lyposomal injury Although there are many hypotheses regarding skin irritation, it is generally believed that surfactants become irritants because they penetrate the stratum corneum which is a "barrier" and then react with the inner cells of the epidermis.

Traditionally, the study of percutaneous absorption has focused on measuring the diffusion of chemicals (for example, surfactants through stratum corneum). Diffusion through an organ as complex as skin and its associated adnexal appendages is challenging to measure and model. Another challenge of cutaneous metabolism is to asses the irritating potential, toxicity, and therapeutic potential of the penetrating compounds.

In vivo, the skin metabolism and percutaneous absorption are very difficult to measure. Finding adequate detection methods and setting up proper experiments are not easy tasks. In vitro studies however are used because of the simplicity of the experimental conditions.

We have obtained information on mildness potentials of the surfactant by carrying out in vitro tests which have been demonstrated to correlate well with in vivo tests.

### In Vitro Zein Solubilization Test

Gotte (E. Gotte, Proc. Int. Cong. Surface Active Subs., 4th Brussels [1964], 3, 83-90) and Schwinger (M.J. Schwinger, Kolloid-Z.Z.Poly., [1969], 233, 898) have shown that a surfactant's ability to solubilize zein, an insoluble maize protein, correlates well with surfactant irritation potential. Specifically, the lower the amount of zein protein dissolved, the milder a surfactant is Conversely, the more zein dissolved, the more irritating the surfactant is.

In order to test irritancy potential, a 1 wt% solution of surfactant (30 mls) was added to 1.5 g zein and stirred at room temperature for 1 hr. Residual zein was collected and dried to constant weight. Differences between starting and residual weights were used to calculate % zein dissolved.

Using the zein solubilization assay, the following results were obtained.

### Zein solubilization Assay

| Surfactant | pH | % Zein Dissolved |
|---|---|---|
| SDS | 6-7 | 86 |
| Blank | 7 | 9 |
| SC10 GAL | 9 | 9 |
| SC12 GAL | 7 and 9 | 26 |
| SC14 GAL | 7 and 9 | 18 |
| SC16 GAL | 9 | Insoluble |

As indicated in the table above, SAGAL salts at pH 7 or 9 dissolve very little zein (9-26%) and thus are potentially mild to the skin.

### EXAMPLE 13

### Use of Galacturonic Acid Derived Surfactants as Coactives in Detergent Bar Composition

| Ingredients | % by Weight |
|---|---|
| C₈₋₂₄ fatty acid | 5%-60% |
| Galacturonic acid derived surfactant | 0.01-45% |
| Alkyl or aryl sulphate or sulphonate | 0-5% |
| Coactive other than uronic acid derivative | 0-50% |
| Moisturiser (eg sorbitol or glycerine) | 0.1-10% |
| Water soluble polymer (eg cellulose or polyacrylates) | 0-10% |
| Sequestering agents (eg citrate) | 0.1-0.5% |
| Dyestuff | < 0.1% |
| Optical brighteners | < 0.1% |
| Whitening agents | 0.1-0.4% |
| Fragrance | 0.1-2.0% |
| Water | Balance |

### EXAMPLE 14

### Galacturonic Acid Derived Surfactant is Used in a Facial/Body Cleanser Composition

| Ingredients | % by Weight |
|---|---|
| C₈₋₂₄ fatty acid salt (eg triethanolamine) | 1-45% |
| Galacturonic acid derived surfactant | 10-75% |
| Alkyl or aryl sulphate or sulphonate | 0-20% |
| Coactive surfactant (eg cocoamidobetaine) | 1-15% |
| Moisturiser (eg sorbitol) | 0.1-15% |
| Refattying alcohol | 0.5-5% |
| Water soluble polymer | 0-10% |
| Thickener | 0-15% |
| Conditioner (eg quaternised cellulose) | 0-0.5% |
| Sequestering agents (eg citrate) | 0.1-0.4% |
| Dyestuff | < 0.1% |
| Optical brighteners | < 0.1% |
| Whitening agents | 0.1-0.4% |
| Fragrance | 0.1-3.0% |
| Preservatives | 0-0.2% |
| Water | Balance |

### EXAMPLE 15

### Galacturonic Acid Derived Surfactant is Used in a Toothpaste Composition

| Ingredients | % by Weight |
|---|---|
| Synthetic surfactants (sodium lauryl sulphate) | 1.5% |
| Galacturonic acid derived surfactant | 0.01-10% |
| Alkyl or aryl sulphate or sulphonate | 0-1% |
| Abrasive (eg silic acid/CaCO₃) | 20-55% |
| Active ingredients (for example, pyrophosphates) | 0.1-2% |
| Humectant (glycerine, sorbitol) | 10-45% |
| Thickeners (cellulose derivatives) | 0-3% |
| Sequestering agents (eg citrate) | 0.1-0.4% |
| Flavoring agents | 0.5-2% |
| Sweeteners | < 0.5% |
| Dyestuff | < 0.1% |
| Water | Balance |

## Claims

1. Use as a mild surfactant in a personal product composition of an anionic glycolipid surfactant which is galacturonic acid derivative having one of the formulae 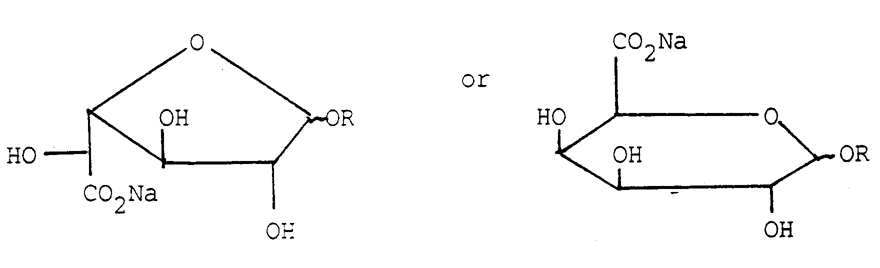 or a glucuronic acid derivative having one of the formulae 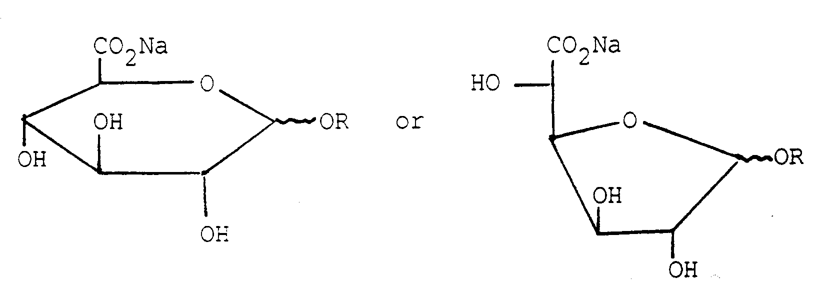 wherein R is a substituted or unsubstituted, saturated or unsaturated, optionally ethoxylated or propoxylated, aliphatic group having 6 to 24 carbon atoms.

2. Use as claimed in claim 1, wherein R is an alkyl group containing from 10 to 16 carbon atoms.

3. Use as claimed in claim 1 or claim 2, in a personal product composition which also comprises a further anionic, nonionic cationic, zwitterionic or amphoteric surfactant.

4. Use as claimed in any one of claims 1 to 3, in a personal product composition which is a toilet bar.

5. Use as claimed in claim 4, in a toilet bar comprising the following:
| Ingredients | % by Weight |
|---|---|
| C₈-C₂₄ fatty acid | 5-60% |
| Galacturonic acid derivative surfactant | 1-45% |
| Alkyl or aryl sulphate or sulphonate | 0-5% |
| Other cosurfactant | 0-50% |
| Sorbitol | 0.1-10% |
| Cellulose | 0-10% |
| Sequestering agent | 0.1-0.5% |
| Water and minors | to balance |

6. Use as claimed in any one of claims 1 to 3, in a personal product composition which comprises a facial/body cleanser, a hair or body shampoo, a conditioner composition, a cosmetic composition or a toothpaste composition.

## Patentansprüche

1. Verwendung eines anionischen Glycolipid-Tensids als mildes Tensid in einer Körperpflege-Zusammensetzung, wobei das Tensid ein Galacturonsäurederivat mit einer der Formeln 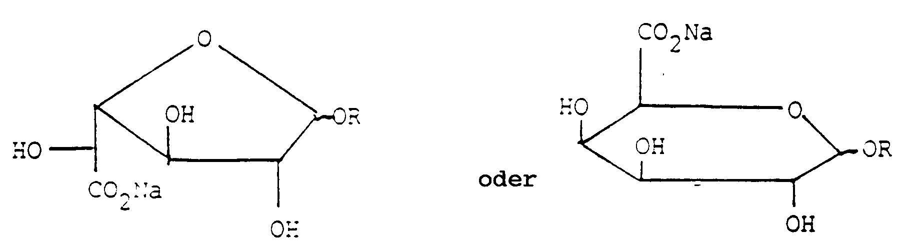 oder ein Glucuronsäurederivat mit einer der Formeln 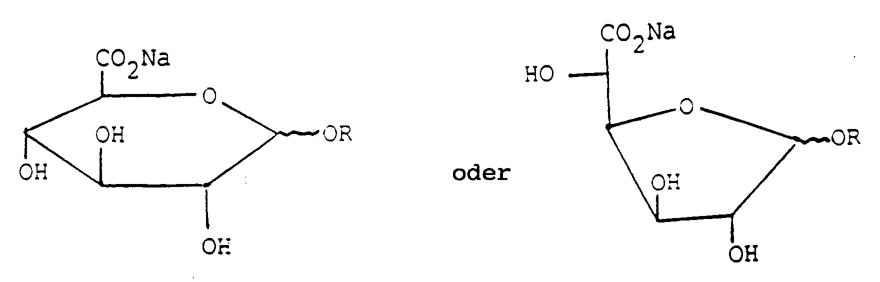 ist, worin R eine substituierte oder unsubstituierte, gesättigte oder ungesättigte, gegebenenfalls ethoxylierte oder propoxylierte aliphatische Gruppe mit 6 bis 24 Kohlenstoffatomen ist.

2. Verwendung nach Anspruch 1, worin R eine Alkylgruppe mit 10 bis 16 Kohlenstoffatomen ist.

3. Verwendung nach einem der Ansprüche 1 oder 2 in einer Körperpflege-Zusammensetzung, die auch weitere anionische, nichtionische, kationische, zwitterionische oder amphotere Tenside enthält.

4. Verwendung nach einem der Ansprüche 1 bis 3 in einer Körperpflege-Zusammensetzung, die eine Stückseife ist.

5. Verwendung nach Anspruch 4 für eine Stückseife, die folgende Inhaltsstoffe umfaßt:
| | |
|---|---|
| Inhaltsstoffe | Gew.-% |
| C₈-C₂₄ Fettsäure | 5 bis 60 % |
| Galacturonsäure-Derivat-Tensid | 1 bis 45 % |
| Alkyl- oder Arylsulfat oder -sulfonat | 0 bis 5 % |
| anderes Co-Tensid | 0 bis 50 % |
| Sorbit | 0,1 bis 10 % |
| Cellulose | 0 bis 10 % |
| Komplexbildner | 0,1 bis 0,5 % |
| Wasser und Bestandteile in geringerem Anteil | Ausgleich |

6. Verwendung nach einem der Ansprüche 1 bis 3 für eine Körperpflege-Zusammensetzung, die ein Gesichts/Körperreinigungsmittel, ein Haar- oder Körpershampoo, eine konditionierende Zusammensetzung, eine kosmetische Zusammensetzung oder eine Zahnpasta-Zusammensetzung umfaßt.

## Revendications

1. Utilisation d'un tensioactif doux dans une composition de produit de soin personnel d'un tensioactif glycolipidique anionique qui est un dérivé d'acide galacturonique ayant l'une des formules 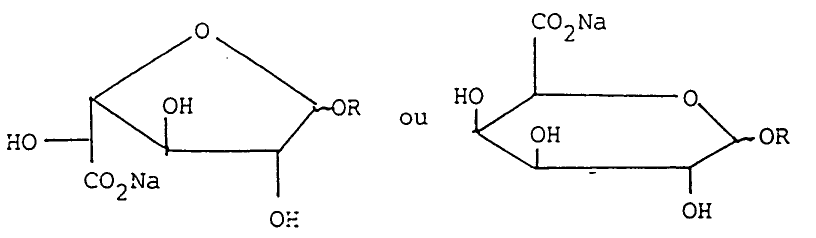 ou un dérivé d'acide glucuronique ayant l'une des formules 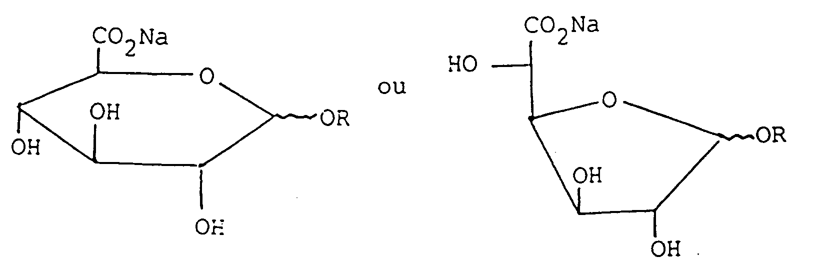 où R est un groupe aliphatique sature ou insaturé, substitué ou non substitué, éventuellement éthoxylé ou propoxylé ayant 6 à 24 atomes de carbone.

2. Utilisation selon la revendication 1, où R est un groupe alkyle contenant de 10 à 16 atomes de carbone.

3. Utilisation selon la revendication 1 ou la revendication 2 dans une composition de produit de soin personnel qui comprend aussi un autre tensioactif anionique, non ionique, cationique, zwitterionique ou amphotère.

4. Utilisation selon l'une quelconque des revendications 1 à 3 dans une composition de produit de soin personnel qui est un savon de toilette.

5. Utilisation selon la revendication 4, dans un savon de toilette comprenant les produits suivants :
| Ingrédients | % en poids |
|---|---|
| Acide gras C₈₋₂₄ | 5 - 60% |
| Tensioactif dérivé de l'acide galacturonique | 1 - 45% |
| Alkyl- ou arylsulfate ou sulfonate | 0 - 5% |
| Autre co-tensioactif | 0 - 50% |
| Sorbitol | 0,1 - 10% |
| Cellulose | 0 - 10% |
| Agent séquestrant | 0,1 - 0,5% |
| Eau et composants minoritaires complément à 100. | |

6. Utilisation selon l'une quelconque des revendications 1 à 3 dans une composition de produit de soin personnel qui comprend un nettoyant pour le visage et le corps, un shampooing pour les cheveux ou le corps, une composition de lotion, une composition cosmétique ou une composition de pâte dentifrice.
